# DEMANDE DE BREVET EUROPEEN

(11) **EP 3 594 851 A1**
(43) Date de publication de la demande: **15.01.2020**
(21) Numéro de dépôt: 19185876.0
(22) Date de dépôt: 11.07.2019
(51) Int. Cl.: G06K 9/00, G06K 9/46

(54) **PROCÉDÉ DE RECONNAISSANCE BIOMÉTRIQUE**

(30) Priorité: 13.07.2018 FR 1856530
(71) Demandeur: Idemia Identity & Security France, 92400 Courbevoie (FR)
(72) Inventeur: MILGRAM, Jonathan, 92400 COURBEVOIE (FR); GENTRIC, Stéphane, 92400 COURBEVOIE (FR); MAURIN, Franck, 92400 COURBEVOIE (FR)
(74) Mandataire: Lavialle, Bruno François Stéphane

(57) **Abrégé**

Procédé de reconnaissance biométrique, comprenant l'étape de calculer un score de similarité d'un vecteur biométrique candidat avec un vecteur biométrique de référence. Au moins l'un des vecteurs biométriques est extrait au moyen d'au moins un réseau de neurones.

## Description

La présente invention concerne le domaine de la reconnaissance biométrique, par exemple à des fins d'identification d'un individu ou de vérification du droit de l'individu à accéder à un lieu ou à des informations.

### Arrière-plan technologique

Classiquement, un procédé de reconnaissance biométrique comprend les étapes de :
- capturer une image du visage d'un candidat à la reconnaissance ;
- extraire de cette image des caractéristiques biométriques sous la forme d'un vecteur biométrique du candidat ;
- calculer un score de similarité du vecteur biométrique du candidat avec un vecteur biométrique de référence mémorisé dans une base de données hébergée par un ordinateur ou dans un support de données détenu par le candidat, comme un circuit intégré incorporé à une carte de données ou à un document d'identité tel qu'un passeport ;
- valider ou refuser la reconnaissance en fonction du score de similarité.

Le vecteur biométrique de référence est obtenu lors d'une opération d'enrôlement qui comprend les étapes de :
- capturer une image du visage d'un candidat à l'enrôlement ;
- extraire de cette image des caractéristiques biométriques sous la forme d'un vecteur biométrique de référence mémorisé dans la base de données hébergée par un ordinateur ou dans le support de données détenu par le candidat.

Actuellement, le vecteur biométrique de référence occupe une place relativement importante dans la mémoire du support de données alors que la taille de cette mémoire est relativement limitée.

En particulier, il ressort du document « Ongoing Face Recognition Vendor Test », P. Grother et al., National Institute of Standards and Technology, https://www.nist.gov/sites/default/files/documents/2019/0 4/04/frvt report 2019 04 04.pdf, que la taille des vecteurs biométriques de visage doit être relativement grande pour atteindre les performances attendues en matière de reconnaissance biométrique.

### Objet de l'invention

Un but de l'invention est de fournir un moyen pour remédier au problème précité ou, plus généralement, limiter au moins en partie les ressources nécessaires à une reconnaissance biométrique.

### Bref exposé de l'invention

A cet effet, on prévoit, selon l'invention, un procédé de reconnaissance biométrique, comprenant les étapes de :
- capturer une image d'une partie du corps d'un candidat à la reconnaissance ;
- extraire de cette image des caractéristiques biométriques sous la forme d'un vecteur biométrique candidat ;
- calculer un score de similarité du vecteur biométrique candidat avec un vecteur biométrique de référence mémorisé ;
- valider ou refuser la reconnaissance en fonction du score de similarité ;
le vecteur biométrique de référence ayant été obtenu lors d'une phase préalable d'enrôlement en :
- capturant une image d'une partie du corps d'un candidat à l'enrôlement ;
- extrayant de cette image des caractéristiques biométriques sous la forme d'un vecteur biométrique de référence.
L'extraction est réalisée au moyen d'au moins un réseau de neurones pour au moins l'un des vecteurs biométriques.

L'utilisation d'un réseau de neurones combinée à une quantification pour créer un vecteur biométrique de visage permet de définir la taille du vecteur biométrique en fonction des performances souhaitées.

Selon un mode de réalisation préféré :
- le réseau de neurones a une couche de sortie comprenant au plus 128 neurones ;
- le vecteur biométrique obtenu par le réseau de neurones subit, avant sa mémorisation, une opération de quantification inférieure ou égale à quatre bits par valeur ;
- le vecteur biométrique quantifié a une taille inférieure à 200 octets autorisant une reconnaissance avec un taux de faux-rejets d'au plus 3% environ et un taux de fausse acceptance d'au plus 0,0001% environ.

L'invention a également pour objet un support de données obtenu par la mise en oeuvre de ce procédé.

D'autres caractéristiques et avantages de l'invention ressortiront à la lecture de la description qui suit d'un mode de mise en oeuvre particulier non limitatif de l'invention.

### Brève description des dessins

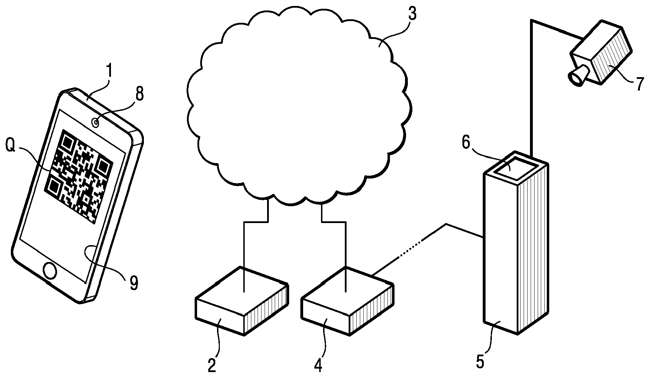

Il sera fait référence à la figure unique annexée représentant schématiquement en perspective un dispositif pour la mise en oeuvre de l'invention.

### Exposé détaillé de l'invention

L'invention est ici décrite en application au contrôle d'accès à un avion dans un aéroport. L'accès à l'avion suppose de disposer d'une carte d'embarquement sur laquelle figurent au moins :
- des éléments d'identification de l'avion et du vol ;
- les nom et prénoms du passager.

La carte d'embarquement est traditionnellement délivrée par une borne automatique à partir d'un numéro de réservation ou toute autre information d'identification, ou au guichet de la compagnie aérienne qui a affrété ou possède l'avion.

Dans le mode de mise en oeuvre qui va être décrit, un vecteur biométrique de référence est associé à un document personnel et plus particulièrement ici, à la carte d'embarquement.

Ceci est parfaitement réalisable avec une carte d'embarquement « papier » mais, dans la mise en oeuvre ici décrite, la carte d'embarquement est dématérialisée.

A cette fin, et en référence à la figure, le passager possédant un téléphone mobile 1 de type « ordiphone » ou « smartphone » se voit proposer le téléchargement d'une application permettant d'éditer sa carte d'embarquement. Cette application est téléchargée depuis un serveur informatique 2 appartenant à la compagnie aérienne possédant ou ayant affrété l'avion. Le serveur informatique 2 comprend de façon classique un processeur et une mémoire contenant des programmes, et est relié à un réseau de transport de données 3, tel que le réseau Internet, auquel le téléphone mobile 1 peut se connecter.

Le téléphone mobile 1 est de type classique et comprend un processeur, une mémoire contenant des programmes (dont certains sont appelés couramment applications), et au moins un capteur d'image 8.

Le serveur informatique 2 exécute un programme comprenant des instructions pour la vente de billets d'avions. Pour acheter un billet d'avion, le futur passager doit s'identifier sur le serveur informatique 2, sélectionner le vol souhaité, puis payer. Le programme lui propose alors de télécharger l'application sur son téléphone mobile 1 afin qu'il puisse créer sa carte d'embarquement.

Lorsque l'application téléchargée est exécutée, elle commande la réalisation d'une connexion du téléphone mobile 1 au serveur informatique 2 pour :
- exécuter un processus d'authentification du passager (saisie d'un identifiant et d'un mot de passe) puis,
- si l'authentification est réussie, exécuter un processus de création de la carte d'embarquement.

Le processus de création de la carte d'embarquement comprend les étapes de :
- capturer une image d'une partie du corps d'un candidat à l'enrôlement ;
- extraire de cette image des caractéristiques biométriques sous la forme d'un vecteur biométrique de référence dit initial ;
- quantifier le vecteur biométrique de référence initial pour obtenir un vecteur biométrique de référence dit quantifié ;
- former à partir du vecteur biométrique de référence quantifié et d'un identifiant du vol un code-barres à deux dimensions.

Pour effectuer la capture d'image, l'application pilote le capteur d'image 8 du téléphone mobile 1 et demande au futur passager de prendre une photographie de son visage (couramment appelée « selfie »). L'application peut afficher à cette fin des indications textuelles ou graphiques permettant au futur passager de prendre une photographie de qualité suffisante (en terme d'exposition, de netteté, de contraste de l'image, de dimensions du visage sur l'image...) pour permettre l'extraction des caractéristiques biométriques.

L'application transfère ensuite, via le réseau de transport de données 3, l'image capturée au serveur informatique 2 dont le programme est agencé pour détecter la position du visage dans l'image, aligner le visage dans l'image, puis pour effectuer l'extraction des caractéristiques biométriques. La détection de la position et l'alignement du visage permettent d'obtenir une image de dimensions prédéterminées recadrée sur le visage : ces opérations sont connues en elles-mêmes et ne seront pas plus détaillées ici.

L'extraction est effectuée par au moins un réseau de neurones entraîné par apprentissage profond. Le réseau de neurones est ici un réseau à convolution qui est paramétré pour fournir un vecteur de référence ayant une taille maximale de 512 octets. Un seul réseau de neurones est ici utilisé mais il est possible d'utiliser plusieurs réseaux de neurones pour faire l'extraction.

De préférence, le réseau de neurones a ici une couche de sortie comprenant au plus 128 neurones pour fournir un vecteur biométrique de référence initial (couramment appelé « template biométrique ») comprenant 128 coordonnées en valeur flottante.

Le vecteur biométrique de référence initial qui est ensuite quantifié pour former le vecteur biométrique de référence quantifié.

La quantification est inférieure ou égale à quatre bits par valeur. On rappelle que la quantification consiste à diviser l'ensemble des valeurs flottantes possibles en intervalles à chacun desquels est affectée une valeur de quantification ici sur quatre bits au maximum, et de comparer chaque valeur flottante du vecteur biométrique de référence initial aux intervalles : la valeur flottante est alors remplacée par la valeur de quantification associée à l'intervalle auquel appartient la valeur flottante. La quantification est ici réalisée sur quatre bits de sorte que le vecteur biométrique quantifié a une taille de 512 bits (128 valeurs flottantes fois quatre bits de la valeur de quantification remplaçant chaque valeur flottante) soit 64 octets. En variante, les intervalles peuvent être différents pour chaque coordonnée.

Le réseau de neurones et l'opération de quantification sont ainsi agencée pour que le vecteur biométrique quantifié ait une taille inférieure à 200 octets et autorise une reconnaissance avec un taux de faux-rejets d'au plus 3% environ et un taux de fausse acceptance d'au plus 0,0001% environ.

Le code-barres à deux dimensions (couramment appelé « QR code » et symbolisé en Q sur la figure) est formé de manière classique sauf en ce que les informations à y faire figurer sont le vecteur biométrique de référence quantifié et une signature (la signature a ici une taille de 16 octets). La signature est une valeur chiffrée correspondant ici au nom du passager et aux informations du vol (notamment l'identifiant du vol). De préférence, on effectuera, préalablement à la formation du code-barres, un hachage du vecteur biométrique de référence quantifié et de la signature. Le code-barres est ensuite renvoyé par le serveur informatique 2 à l'application. Le code-barre est ici agencé pour contenir également : un nom et éventuellement un prénom du candidat à l'enrôlement ; une ou des informations relatives à un trajet que doit accomplir le candidat à l'enrôlement (numéro de vol, porte d'embarquement, destination, date et heure de départ, numéro de train, gare de départ, gare d'arrivée...).

On notera que, après la capture, tout ou partie des opérations permettant la constitution du code-barres à deux dimensions peut être indifféremment réalisée au sein du téléphone mobile 1 ou du serveur informatique 2, et notamment :
- l'extraction des caractéristiques biométriques peut être réalisée par l'application au sein du téléphone mobile 1 ou par le serveur informatique 2 après transmission de l'image capturée ;
- de même pour la formation du code-barres.

A l'aéroport, le passager est censé lancer l'exécution de l'application de manière à permettre l'affichage de la carte virtuelle d'embarquement. L'application est également agencée pour présenter, sur demande du passager, le code-barres mais aussi d'autres informations concernant le vol comme notamment l'heure de départ. L'application est en outre, de préférence, agencée pour se connecter au serveur informatique 2 de la compagnie aérienne ou à un serveur informatique 4 dédié de l'aéroport auquel le serveur informatique 2 est connecté et auquel le serveur informatique 2 a communiqué les informations relatives au passager. Ceci permet d'afficher sur le téléphone mobile 1, par voie de notification ou d'alerte, les informations relatives à l'enregistrement, la porte d'embarquement, les retards éventuels...

Le serveur informatique 4 de l'aéroport est relié à une borne 5 de contrôle d'accès à la zone d'embarquement. La borne 5 comprend un processeur et une mémoire pour exécuter un procédé de commande, ainsi qu'un lecteur 6 de code-barres à deux dimensions et au moins une caméra 7 agencée pour capturer des images du visage des passagers se présentant devant la borne 5. La borne 5 est agencée pour commander l'ouverture d'un sas d'accès sous certaines conditions.

Pour accéder à la zone d'embarquement, le passager commande l'application pour que le code-barres soit affiché sur l'écran 9 de son téléphone mobile 1 qu'il présente face au lecteur 6. La borne 5 détecte le code-barres et commande la caméra 7 pour capturer une image du visage du passager.

Le programme de commande de la borne 5 exécute ensuite les étapes de :
- extraire de cette image des caractéristiques biométriques sous la forme d'un vecteur biométrique candidat ;
- lire le code-barres et en extraire le vecteur biométrique de référence quantifié et la signature ;
- calculer un score de similarité du vecteur biométrique candidat avec le vecteur biométrique de référence quantifié et déchiffrer la signature ;
- valider ou refuser la reconnaissance en fonction du score de similarité, de l'exactitude du nom du passager et de l'existence d'un vol à venir correspondant à l'identifiant du vol ;
- ouvrir le sas d'accès si la reconnaissance est validée et émettre une alerte si la reconnaissance est refusée.

Le calcul du score de similarité est une distance entre les deux vecteurs. Plus précisément, le calcul du score de similarité comprend une comparaison de la distance à un seuil défini en fonction d'un taux souhaité de fausse acceptation et d'un taux souhaité de faux rejet.

Selon une première approche, le vecteur biométrique candidat est binarisé avant le calcul du score de similarité. Le score de similarité est ici une distance de Hamming dont le calcul est connu en lui-même.

Le calcul de la distance de Hamming est très rapide. Toutefois, la binarisation du vecteur biométrique candidat dégrade la précision de la reconnaissance.

Selon une deuxième approche, le vecteur biométrique candidat, qui ne contient que des valeurs réelles, reste inchangé (sans quantification), ce qui permet de conserver une bonne précision de la reconnaissance.

Dans une première version, la valeur de chaque composante du vecteur biométrique de référence quantifié qui est égale à 0 est remplacée par -1 pour obtenir un vecteur biométrique de référence quantifié transformé. On calcule alors la distance cosinus séparant le vecteur biométrique de référence quantifié transformé et le vecteur biométrique candidat.

Le score de similarité est alors une distance cosinus dont le calcul est classique.

Dans une deuxième version, on calcule, lors de la conception de l'algorithme de reconnaissance, la valeur moyenne µᵢ et l'écart-type σᵢ de chaque composante de vecteurs biométriques extraits d'un échantillon d'images d'une banque de données de visages. Ensuite, pour chaque composante du vecteur biométrique de référence quantifié :
- si la valeur de la composante i est 0, alors la valeur de la composante est remplacée par xᵢ= µᵢ-σᵢ ;
- si la valeur de la composante i est 1, alors la valeur de la composante est remplacée par xᵢ= yᵢ+ σᵢ.

Le score de similarité est alors une distance cosinus calculée de manière classique.

Bien entendu, l'invention n'est pas limitée au mode de réalisation décrit mais englobe toute variante entrant dans le champ de l'invention telle que définie par les revendications.

En particulier, le vecteur biométrique de référence peut être mémorisé sous forme graphique (comme dans le code-barres à deux dimensions) mais aussi sous forme électronique par exemple dans une mémoire d'un circuit intégré de type RFID contenu dans un document tel qu'un passeport, dans une mémoire d'un circuit intégré de carte à circuit intégré, dans une mémoire d'ordinateur...

Bien que dans le mode de mise en oeuvre décrit, ce soit le vecteur biométrique de référence qui soit fourni par le réseau de neurones, ce peut être le vecteur biométrique candidat. Les deux vecteurs peuvent être obtenus au moyen d'un réseau de neurones et/ou peuvent être quantifiés.

L'extraction peut être réalisée dans le téléphone mobile. On évite ainsi que des données biométriques transitent par un ordinateur distant.

Selon le type ou le nombre de réseaux de neurones utilisés, la taille du vecteur biométrique peut être différente de celle indiquée. Par exemple avec deux ou trois réseaux de neurones, le vecteur biométrique peut avoir une taille de 1024 ou 1536 octets. Un exemple de réseau de neurones et de méthode d'apprentissage utilisable est celui décrit dans le document « DeepVisage : Making face récognition simple yet with powerful generalization skills », Abul Hasnat et al., The IEEE International Conférence on Computer Vision (ICCV), 2017, pp. 1682-1691.

Le code-barres à deux dimensions peut être mémorisé dans la mémoire d'un dispositif électronique telle qu'un téléphone ou une tablette électronique multimédia, ou imprimé sur un document tel qu'une carte d'embarquement « papier ».

Il est possible d'associer au vecteur biométrique de référence mêlé par hachage à l'identifiant du vol également une information de signature qui comprendra par exemple une valeur d'intégrité calculée sur les bits du vecteur biométrique de référence obtenu après hachage.

D'autres informations peuvent être mêlées au vecteur biométrique de référence comme par exemple une date, le nom du passager ou toute autre suite de caractères alphanumériques.

Au lieu d'être réalisé depuis un téléphone mobile, l'enrôlement peut être réalisé depuis une borne d'enregistrement pourvue d'un capteur biométrique et située par exemple à l'aéroport.

En variante, la quantification utilisée est une binarisation. On rappelle que la binarisation est une opération consistant à comparer à un seuil la valeur de chaque composante du vecteur biométrique de référence quantifié, et remplacer la valeur de chaque composante par 0 lorsque la valeur de cette composante est inférieure ou égale au seuil et par 1 lorsque la valeur de cette composante est supérieure au seuil. A titre d'exemple, si le seuil est égal à 0, on remplace par 0 chaque composante inférieure ou égale à 0 et par 1 chaque composante supérieure à 0. Ainsi, si les composantes du vecteur biométrique de référence initial sont codées chacune sur quatre octets, soit 32 bits, et que l'on binarise chacune de ses composantes, le vecteur biométrique de référence quantifié résultant de la binarisation du vecteur biométrique de référence initial est 32 fois plus petite que celle du vecteur biométrique de référence initial. En variante, le seuil peut être différent pour chaque composante. De préférence alors, le seuil correspond à une valeur moyenne de chaque composante. Ces valeurs moyennes ont été calculées, au moment de la conception de l'algorithme de quantification, à partir d'une pluralité de vecteurs biométriques de référence extraits de visages d'une base de données de visages.

D'autres modes de quantification sont possibles. Par exemple, on pourrait diviser l'intervalle des valeurs possibles de chaque valeur du vecteur biométrique de référence initial en quatre sous intervalles et remplacer cette valeur réelle (initialement codée sur 32 bits) par une valeur quantifiée égale à 0, 1, 2 ou 3 selon le sous-intervalle auquel la valeur réelle appartient. La valeur quantifiée est codée sur seulement deux bits.

A l'issue de la quantification, le vecteur biométrique de référence quantifié peut avoir une taille différente de celle mentionnée et par exemple une taille inférieure à 200 octets environ, voire une taille inférieure à 100 octets, voire inférieure à 64 octets et par exemple une taille de 32 octets.

La signature résulte ici d'un codage du nom du candidat à l'enrôlement et d'une information relative à un trajet qu'il doit réaliser. D'autres données peuvent être utilisées pour réaliser la signature.

Toute donnée, sous forme de signature ou pas, peut-être codée dans le code-barre à deux dimensions.

## Revendications

1. Procédé de reconnaissance biométrique, comprenant les étapes de :
- capturer une image du visage d'un candidat à la reconnaissance ;
- extraire de cette image des caractéristiques biométriques sous la forme d'un vecteur biométrique candidat ;
- calculer un score de similarité du vecteur biométrique candidat avec un vecteur biométrique de référence mémorisé ;
- valider ou refuser la reconnaissance en fonction du score de similarité ;
le vecteur biométrique de référence ayant été obtenu lors d'une phase préalable d'enrôlement en :
- capturant une image du visage d'un candidat à l'enrôlement ;
- extrayant de cette image des caractéristiques biométriques sous la forme d'un vecteur biométrique de référence ;
**caractérisé en ce que** l'extraction est réalisée au moyen d'au moins un réseau de neurones pour au moins l'un des vecteurs biométriques.

2. Procédé selon la revendication 1, dans lequel le réseau de neurones a une couche de sortie comprenant au plus 128 neurones.

3. Procédé selon l'une quelconque des revendications précédentes, dans lequel le vecteur biométrique obtenu par le réseau de neurones subit une opération de quantification avant sa mémorisation.

4. Procédé selon l'une quelconque des revendications précédentes, dans lequel la quantification est inférieure ou égale à quatre bits par coordonnées du vecteur biométrique.

5. Procédé selon la revendication 6, dans lequel la quantification est une binarisation.

6. Procédé selon l'une quelconque des revendications 3 à 5 6, dans lequel le vecteur biométrique quantifié a une taille inférieure à 200 octets et autorise une reconnaissance avec un taux de faux-rejets d'au plus 3% environ et un taux de fausse acceptance d'au plus 0.0001% environ.

7. Procédé selon la revendication 6, dans lequel la quantification est réalisée pour fournir un vecteur biométrique ayant une taille inférieure à 100 octets, de préférence inférieure à 64 octets, voire égale à 32 octets.

8. Procédé selon l'une quelconque des revendications 5 à 7, dans lequel le vecteur biométrique quantifié est mémorisé sous la forme d'un code-barres à deux dimensions.

9. Procédé selon la revendication 8, dans lequel le vecteur biométrique quantifié est associé à une signature avant d'être transformé en code-barres à deux dimensions.

10. Procédé selon la revendication 8 ou la revendication 9, dans lequel le code-barre est agencé pour contenir également au moins l'une des données suivantes :
- un nom du candidat à l'enrôlement ;
- un prénom du candidat à l'enrôlement ;
- une information relative à un trajet que doit accomplir le candidat à l'enrôlement.

11. Procédé selon l'une quelconque des revendications précédentes, dans lequel le calcul du score de similarité comprend un calcul de distance entre les deux vecteurs biométriques et une comparaison de la distance à un seuil défini en fonction d'un taux souhaité de fausse acceptation et d'un taux souhaité de faux rejet.

12. Procédé selon la revendication 11, dans lequel la distance est une distance cosinus ou une distance de Hamming.

13. Support personnel de données à des fins de reconnaissance biométrique de visage, le support personnel comprenant un élément de mémorisation d'un vecteur biométrique de référence ayant une taille inférieure à 200 octets environ, de préférence inférieure à 100 octets, voire inférieure à 64 octets, voire égale à 32 octets.

14. Support selon la revendication 13, dans lequel l'élément de mémorisation est un code-barre à deux dimensions et contient également au moins l'une des données suivantes :
- une signature associée au vecteur biométrique de référence,
- un nom du candidat à l'enrôlement ;
- un prénom du candidat à l'enrôlement ;
- une information relative à un trajet que doit accomplir le candidat à l'enrôlement.
